# EUROPEAN PATENT SPECIFICATION

(11) **EP 3 235 812 B1**
(45) Date of publication and mention of the grant of the patent: **04.09.2019**
(21) Application number: 17160386.3
(22) Date of filing: 17.05.2012
(51) Int. Cl.: C07D 215/56, C07B 59/00, A61K 31/47, A61P 11/12

(54) **DEUTERATED DERIVATIVES OF IVACAFTOR**
DEUTERIERTE IVACAFTOR-DERIVATE
DÉRIVÉS DEUTÉRÉS D'IVACAFTOR

(30) Priority: 18.05.2011 US 201161487497 P
(43) Date of publication of application: 25.10.2017
(62) Divisional of application: 12725197.3
(73) Proprietor: Vertex Pharmaceuticals (Europe) Limited, London W2 6BD (GB)
(72) Inventor: Morgan,, Adam J, Ashland, MA Massachusetts 01721 (US)
(74) Representative: Oates, Edward Christopher

(56) References cited:
- WO-A2-95/26325
- WO-A2-2006/002421
- "Ivacaftor (VX-770). Application Number NDA 203188Orig1s000: Clinical Pharmacology and Biopharmaceutics Review(s)", U.S. FDA, Center for Drug Evaluation and Research , 18 January 2012 (2012-01-18), XP055031552, Retrieved from the Internet: URL:http://www.accessdata.fda.gov/drugsatf da_docs/nda/2012/203188Orig1s000ClinPharmR .pdf [retrieved on 2012-07-03]
- O'DRISCOLL: "Heavyweight drugs", CHEMISTRY & INDUSTRY, 9 March 2009 (2009-03-09), pages 24-26, XP002636700, ISSN: 0009-3068
- CONCERT PHARMACEUTICALS: "Precision Deuterium Chemistry Backgrounder", INTERNET CITATION, 2007, pages 1-6, XP002636701, Retrieved from the Internet: URL:http://www.webcitation.org/5e81SGCnl [retrieved on 2011-05-12]
- BUTEAU: "Deuterated Drugs: Unexpectedly Nonobvious?", JOURNAL OF HIGH TECHNOLOGY LAW, SUFFOLK UNIVERSITY LAW SCHOOL, US , vol. X, no. 1 1 January 2009 (2009-01-01), pages 22-74, XP002636702, ISSN: 1536-7983 Retrieved from the Internet: URL:http://www.law.suffolk.edu/highlights/ stuorgs/jhtl/docs/pdf/Buteau_10JHTL1.pdf [retrieved on 2009-01-01]
- Simpson: "European Patent Application 12725197: Response to communication dated 7 January 2014.", European Patent Register, July 2014 (2014-07), pages 1-6, XP055389598, Retrieved from the Internet: URL:https://register.epo.org/application?d ocumentId=EWB4MILQ1188235&number=EP1272519 7&lng=en&npl=false [retrieved on 2017-07-11]

## Description

### Background of the Invention

Many current medicines suffer from poor absorption, distribution, metabolism and/or excretion (ADME) properties that prevent their wider use or limit their use in certain indications. Poor ADME properties are also a major reason for the failure of drug candidates in clinical trials. While formulation technologies and prodrug strategies can be employed in some cases to improve certain ADME properties, these approaches often fail to address the underlying ADME problems that exist for many drugs and drug candidates. One such problem is rapid metabolism that causes a number of drugs, which otherwise would be highly effective in treating a disease, to be cleared too rapidly from the body. A possible solution to rapid drug clearance is frequent or high dosing to attain a sufficiently high plasma level of drug. This, however, introduces a number of potential treatment problems such as poor patient compliance with the dosing regimen, side effects that become more acute with higher doses, and increased cost of treatment. A rapidly metabolized drug may also expose patients to undesirable toxic or reactive metabolites.

Another ADME limitation that affects many medicines is the formation of toxic or biologically reactive metabolites. As a result, some patients receiving the drug may experience toxicities, or the safe dosing of such drugs may be limited such that patients receive a suboptimal amount of the active agent. In certain cases, modifying dosing intervals or formulation approaches can help to reduce clinical adverse effects, but often the formation of such undesirable metabolites is intrinsic to the metabolism of the compound.

In some select cases, a metabolic inhibitor will be co-administered with a drug that is cleared too rapidly. Such is the case with the protease inhibitor class of drugs that are used to treat HIV infection. The FDA recommends that these drugs be co-dosed with ritonavir, an inhibitor of cytochrome P450 enzyme 3A4 (CYP3A4), the enzyme typically responsible for their metabolism (see Kempf, D.J. et al., Antimicrobial agents and chemotherapy, 1997, 41(3): 654-60). Ritonavir, however, causes adverse effects and adds to the pill burden for HIV patients who must already take a combination of different drugs. Similarly, the CYP2D6 inhibitor quinidine has been added to dextromethorphan for the purpose of reducing rapid CYP2D6 metabolism of dextromethorphan in a treatment of pseudobulbar affect. Quinidine, however, has unwanted side effects that greatly limit its use in potential combination therapy (see Wang, L et al., Clinical Pharmacology and Therapeutics, 1994, 56(6 Pt 1): 659-67; and FDA label for quinidine at www.accessdata.fda.gov).

In general, combining drugs with cytochrome P450 inhibitors is not a satisfactory strategy for decreasing drug clearance. The inhibition of a CYP enzyme's activity can affect the metabolism and clearance of other drugs metabolized by that same enzyme. CYP inhibition can cause other drugs to accumulate in the body to toxic levels.

A potentially attractive strategy for improving a drug's metabolic properties is deuterium modification. In this approach, one attempts to slow the CYP-mediated metabolism of a drug or to reduce the formation of undesirable metabolites by replacing one or more hydrogen atoms with deuterium atoms. Deuterium is a safe, stable, nonradioactive isotope of hydrogen. Compared to hydrogen, deuterium forms stronger bonds with carbon. In select cases, the increased bond strength imparted by deuterium can positively impact the ADME properties of a drug, creating the potential for improved drug efficacy, safety, and/or tolerability. At the same time, because the size and shape of deuterium are essentially identical to those of hydrogen, replacement of hydrogen by deuterium would not be expected to affect the biochemical potency and selectivity of the drug as compared to the original chemical entity that contains only hydrogen.

Over the past 35 years, the effects of deuterium substitution on the rate of metabolism have been reported for a very small percentage of approved drugs (see, e.g., Blake, MI et al, J Pharm Sci, 1975, 64:367-91; Foster, AB, Adv Drug Res, 1985, 14:1-40 ("Foster"); Kushner, DJ et al, Can J Physiol Pharmacol, 1999, 79-88; Fisher, MB et al, Curr Opin Drug Discov Devel, 2006, 9:101-09 ("Fisher")). The results have been variable and unpredictable. For some compounds deuteration caused decreased metabolic clearance *in vivo.* For others, there was no change in metabolism. Still others demonstrated increased metabolic clearance. The variability in deuterium effects has also led experts to question or dismiss deuterium modification as a viable drug design strategy for inhibiting adverse metabolism (see Foster at p. 35 and Fisher at p. 101).

The effects of deuterium modification on a drug's metabolic properties are not predictable even when deuterium atoms are incorporated at known sites of metabolism. Only by actually preparing and testing a deuterated drug can one determine if and how the rate of metabolism will differ from that of its non-deuterated counterpart. *See,* for example, Fukuto et al. (J. Med. Chem., 1991, 34, 2871-76). Many drugs have multiple sites where metabolism is possible. The site(s) where deuterium substitution is required and the extent of deuteration necessary to see an effect on metabolism, if any, will be different for each drug.

This invention relates to a pharmaceutical composition comprising novel derivatives of ivacaftor, and pharmaceutically acceptable salts thereof, as defined in claim 1. This invention also provides a pharmaceutical composition for use in treating cystic fibrosis, as defined in claim 13. The invention also provides a pharmaceutical composition for use in treating chronic obstructive pulmonary disease (COPD), as defined in claim 14.

Ivacaftor, also known as VX-770 and by the chemical name, N-(2,4-di-tert-butyl-5-hydroxyphenyl)-4-oxo-1,4-dihydroquinoline-3-carboxamide, acts as a CFTR potentiator. Results from phase III trials of VX-770 in patients with cystic fibrosis carrying at least one copy of the G551D-CFTR mutation demonstrated marked levels of improvement in lung function and other key indicators of the disease including sweat chloride levels, likelihood of pulmonary exacerbations and body weight. VX-770 is also currently in phase II clinical trials in combination with VX-809 (a CFTR corrector) for the oral treatment of cystic fibrosis patients who carry the more common ΔF508-CFTR mutation. VX-770 was granted fast track designation and orphan drug designation by the FDA in 2006 and 2007, respectively.

Despite the beneficial activities of VX-770, there is a continuing need for new compounds to treat the aforementioned diseases and conditions.

### Brief Description of the Drawings

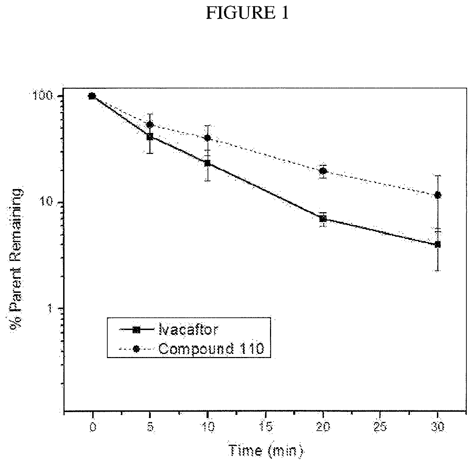

Figure 1 depicts the percentage of compound remaining over time for Compound **110** and for ivacaftor in human cytochrome P450-specific SUPERSOMES™.

### Definitions

The term "treat" means decrease, suppress, attenuate, diminish, arrest, or stabilize the development or progression of a disease (e.g., a disease or disorder delineated herein), lessen the severity of the disease or improve the symptoms associated with the disease.

"Disease" means any condition or disorder that damages or interferes with the normal function of a cell, tissue, or organ.

It will be recognized that some variation of natural isotopic abundance occurs in a synthesized compound depending upon the origin of chemical materials used in the synthesis. Thus, a preparation of VX-770 will inherently contain small amounts of deuterated isotopologues. The concentration of naturally abundant stable hydrogen and carbon isotopes, notwithstanding this variation, is small and immaterial as compared to the degree of stable isotopic substitution of compounds disclosed herein. See, for instance, Wada, E et al., Seikagaku, 1994, 66:15; Gannes, LZ et al., Comp Biochem Physiol Mol Integr Physiol, 1998, 119:725.

In the compounds disclosed herein any atom not specifically designated as a particular isotope is meant to represent any stable isotope of that atom. Unless otherwise stated, when a position is designated specifically as "H" or "hydrogen", the position is understood to have hydrogen at its natural abundance isotopic composition. Also unless otherwise stated, when a position is designated specifically as "D" or "deuterium", the position is understood to have deuterium at an abundance that is at least 3000 times greater than the natural abundance of deuterium, which is 0.015% (i.e., at least 45% incorporation of deuterium).

The term "isotopic enrichment factor" as used herein means the ratio between the isotopic abundance and the natural abundance of a specified isotope.

In the invention, the isotopic enrichment factor for each designated deuterium atom is at least 6000. Other compounds disclosed herein have an isotopic enrichment factor for each designated deuterium atom of at least 3500 (52.5% deuterium incorporation at each designated deuterium atom), at least 4000 (60% deuterium incorporation), at least 4500 (67.5% deuterium incorporation), at least 5000 (75% deuterium), at least 5500 (82.5% deuterium incorporation), at least 6000 (90% deuterium incorporation), at least 6333.3 (95% deuterium incorporation), at least 6466.7 (97% deuterium incorporation), at least 6600 (99% deuterium incorporation), or at least 6633.3 (99.5% deuterium incorporation).

The term "isotopologue" refers to a species in which the chemical structure differs from a specific compound disclosed herein only in the isotopic composition thereof.

The term "compound," when referring to a compound disclosed herein, refers to a collection of molecules having an identical chemical structure, except that there may be isotopic variation among the constituent atoms of the molecules. Thus, it will be clear to those of skill in the art that a compound represented by a particular chemical structure containing indicated deuterium atoms, will also contain lesser amounts of isotopologues having hydrogen atoms at one or more of the designated deuterium positions in that structure. The relative amount of such isotopologues in a compound disclosed herein will depend upon a number of factors including the isotopic purity of deuterated reagents used to make the compound and the efficiency of incorporation of deuterium in the various synthesis steps used to prepare the compound. However, as set forth above the relative amount of such isotopologues *in toto* will be less than 49.9% of the compound. In other embodiments, the relative amount of such isotopologues *in toto* will be less than 47.5%, less than 40%, less than 32.5%, less than 25%, less than 17.5%, less than 10%, less than 5%, less than 3%, less than 1%, or less than 0.5% of the compound.

Disclosed herein are salts of the compounds disclosed herein. A salt of a compound disclosed herein is formed between an acid and a basic group of the compound, such as an amino functional group, or a base and an acidic group of the compound, such as a carboxyl functional group. According to another embodiment, the compound is a pharmaceutically acceptable acid addition salt.

The term "pharmaceutically acceptable," as used herein, refers to a component that is, within the scope of sound medical judgment, suitable for use in contact with the tissues of humans and other mammals without undue toxicity, irritation, allergic response and the like, and are commensurate with a reasonable benefit/risk ratio. A "pharmaceutically acceptable salt" means any non-toxic salt that, upon administration to a recipient, is capable of providing, either directly or indirectly, a compound disclosed herein. A "pharmaceutically acceptable counterion" is an ionic portion of a salt that is not toxic when released from the salt upon administration to a recipient.

Acids commonly employed to form pharmaceutically acceptable salts include inorganic acids such as hydrogen bisulfide, hydrochloric acid, hydrobromic acid, hydroiodic acid, sulfuric acid and phosphoric acid, as well as organic acids such as paratoluenesulfonic acid, salicylic acid, tartaric acid, bitartaric acid, ascorbic acid, maleic acid, besylic acid, fumaric acid, gluconic acid, glucuronic acid, formic acid, glutamic acid, methanesulfonic acid, ethanesulfonic acid, benzenesulfonic acid, lactic acid, oxalic acid, para-bromophenylsulfonic acid, carbonic acid, succinic acid, citric acid, benzoic acid and acetic acid, as well as related inorganic and organic acids. Such pharmaceutically acceptable salts thus include sulfate, pyrosulfate, bisulfate, sulfite, bisulfite, phosphate, monohydrogenphosphate, dihydrogenphosphate, metaphosphate, pyrophosphate, chloride, bromide, iodide, acetate, propionate, decanoate, caprylate, acrylate, formate, isobutyrate, caprate, heptanoate, propiolate, oxalate, malonate, succinate, suberate, sebacate, fumarate, maleate, butyne-1,4-dioate, hexyne-1,6-dioate, benzoate, chlorobenzoate, methylbenzoate, dinitrobenzoate, hydroxybenzoate, methoxybenzoate, phthalate, terephthalate, sulfonate, xylene sulfonate, phenylacetate, phenylpropionate, phenylbutyrate, citrate, lactate, β-hydroxybutyrate, glycolate, maleate, tartrate, methanesulfonate, propanesulfonate, naphthalene-1-sulfonate, naphthalene-2-sulfonate, mandelate and other salts. In one embodiment, pharmaceutically acceptable acid addition salts include those formed with mineral acids such as hydrochloric acid and hydrobromic acid, and especially those formed with organic acids such as maleic acid.

The term "stable compounds," as used herein, refers to compounds which possess stability sufficient to allow for their manufacture and which maintain the integrity of the compound for a sufficient period of time to be useful for the purposes detailed herein (e.g., formulation into therapeutic products, intermediates for use in production of therapeutic compounds, isolatable or storable intermediate compounds, treating a disease or condition responsive to therapeutic agents).

"D" and "d" both refer to deuterium. "Stereoisomer" refers to both enantiomers and diastereomers. "Tert" and "t-" each refer to tertiary. "US" refers to the United States of America.

"Substituted with deuterium" refers to the replacement of one or more hydrogen atoms with a corresponding number of deuterium atoms.

Throughout this specification, a variable may be referred to generally (e.g., "each R") or may be referred to specifically (e.g., R¹, R², R³, etc.). Unless otherwise indicated, when a variable is referred to generally, it is meant to include all specific embodiments of that particular variable.

### Therapeutic Compounds

The present invention provides a pharmaceutical composition suitable for oral administration comprising a compound of Formula I: 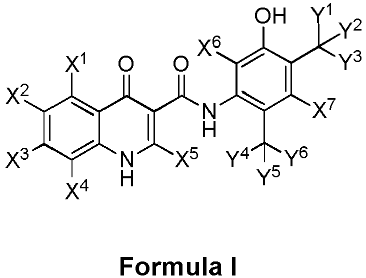 or a pharmaceutically acceptable salt thereof, wherein
each of X¹, X², X³, X⁴, X⁵, X⁶, and X⁷ is independently hydrogen or deuterium;
each of Y¹, Y², and Y³ are CD₃;
each of Y⁴, Y⁵, and Y⁶ is independently CH₃ or CD₃;
wherein the isotopic enrichment factor for each designated deuterium atom is at least 6000; and wherein any atom not designated as deuterium is present at its natural isotopic abundance, provided that the compound of Formula I is not compound 100: 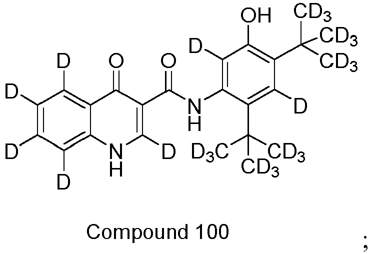 and a pharmaceutically acceptable carrier.

In one embodiment, X¹, X², X³, and X⁴ are the same. In one aspect of this embodiment, X⁶ and X⁷ are the same. In one aspect of this embodiment, Y⁴, Y⁵, and Y⁶ are the same.. In a more particular example, X⁶ and X⁷ are the same.

In one aspect of this embodiment, each of Y⁴, Y⁵, and Y⁶ is the same. In one example of this aspect, X⁶ and X⁷ are the same.

Y¹, Y², and Y³ are CD₃. In one aspect of this embodiment, Y⁴, Y⁵, and Y⁶ are CH₃. In another embodiment, Y⁴, Y⁵, and Y⁶ are CD₃. In yet another embodiment, Y¹, Y², Y³, Y⁴, Y⁵, and Y⁶ are CD₃.

In one embodiment, each of Y⁴, Y⁵, and Y⁶ is the same. In one aspect of this embodiment, X⁶ and X⁷ are the same.

In one example of any of the foregoing embodiments, aspects or examples, X⁵ is hydrogen. In another example, X⁵ is deuterium.

In one embodiment, the compound of Formula I is any one of the compounds of table 1,

**Table 1**

| **Cmpd #** | **X¹** | **X²** | **X³** | **X⁴** | **X⁵** | **X⁶** | **X⁷** | **Y¹** | **Y²** | **Y³** | **Y⁴** | **Y⁵** | **Y⁶** |
|---|---|---|---|---|---|---|---|---|---|---|---|---|---|
| 101 | H | H | H | H | D | H | H | CD₃ | CD₃ | CD₃ | CD₃ | CD₃ | CD₃ |
| 102 | H | H | H | H | D | H | H | CD₃ | CD₃ | CD₃ | CH₃ | CH₃ | CH₃ |
| 105 | H | H | H | H | H | H | H | CD₃ | CD₃ | CD₃ | CD₃ | CD₃ | CD₃ |
| 106 | H | H | H | H | H | H | H | CD₃ | CD₃ | CD₃ | CH₃ | CH₃ | CH₃ |

or a pharmaceutically acceptable salt thereof, wherein any atom not designated as deuterium is present at its natural isotopic abundance.

In one embodiment, the compound of Formula I is any one of the compounds of table 2,

**Table 2**

| **Cmpd #** | **X¹** | **X²** | **X³** | **X⁴** | **X⁵** | **X⁶** | **X⁷** | **Y¹** | **Y²** | **Y³** | **Y⁴** | **Y⁵** | **Y⁶** |
|---|---|---|---|---|---|---|---|---|---|---|---|---|---|
| 110 | H | H | H | H | H | D | D | CD₃ | CD₃ | CD₃ | CD₃ | CD₃ | CD₃ |
| 111 | H | H | H | H | D | D | D | CD₃ | CD₃ | CD₃ | CD₃ | CD₃ | CD₃ |
| 112 | H | H | H | H | D | D | D | CD₃ | CD₃ | CD₃ | CH₃ | CH₃ | CH₃ |
| 115 | H | H | H | H | H | D | D | CD₃ | CD₃ | CD₃ | CH₃ | CH₃ | CH₃ |
| 117 | D | D | D | D | D | D | D | CD₃ | CD₃ | CD₃ | CH₃ | CH₃ | CH₃ |
| 119 | D | D | D | D | D | H | H | CD₃ | CD₃ | CD₃ | CH₃ | CH₃ | CH₃ |
| 121 | H | H | H | H | H | H | D | CD₃ | CD₃ | CD₃ | CD₃ | CD₃ | CD₃ |
| 122 | H | H | H | H | H | H | D | CD₃ | CD₃ | CD₃ | CH₃ | CH₃ | CH₃ |

or a pharmaceutically acceptable salt thereof, wherein any atom not designated as deuterium is present at its natural isotopic abundance.

In one embodiment, the compound of Formula I is any one of the compounds of table 3,

**Table 3**

| **Cmpd #** | **X¹** | **X²** | **X³** | **X⁴** | **X⁵** | **X⁶** | **X⁷** | **Y¹** | **Y²** | **Y³** | **Y⁴** | **Y⁵** | **Y⁶** |
|---|---|---|---|---|---|---|---|---|---|---|---|---|---|
| 301 | D | D | D | D | D | H | H | CD₃ | CD₃ | CD₃ | CD₃ | CD₃ | CD₃ |
| | | | | | | | | | | | | | |
| 304 | D | D | D | D | H | D | D | CD₃ | CD₃ | CD₃ | CD₃ | CD₃ | CD₃ |
| 305 | D | D | D | D | H | H | H | CD₃ | CD₃ | CD₃ | CD₃ | CD₃ | CD₃ |

or a pharmaceutically acceptable salt thereof, wherein any atom not designated as deuterium is present at its natural isotopic abundance.

In another set of embodiments, any atom not designated as deuterium in any of the embodiments, examples or aspects set forth above is present at its natural isotopic abundance.

The synthesis of compounds of Formula I may be readily achieved by synthetic chemists of ordinary skill by reference to the Exemplary Synthesis and Examples disclosed herein. Relevant procedures analogous to those of use for the preparation of compounds of Formula I and intermediates thereof are disclosed, for instance in WO 2007075946, WO 2011019413, WO 2010019239, WO 2007134279, WO 2007079139 and WO 2006002421.

Such methods can be carried out utilizing corresponding deuterated and optionally, other isotope-containing reagents and/or intermediates to synthesize the compounds delineated herein, or invoking standard synthetic protocols known in the art for introducing isotopic atoms to a chemical structure.

### Exemplary Synthesis

A convenient method for synthesizing compounds of Formula I is depicted in Scheme 1.

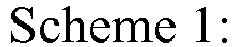

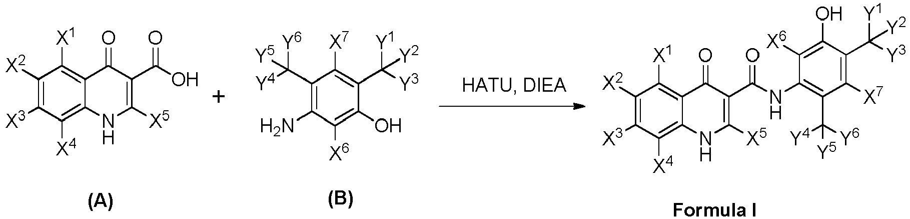

Compounds of **Formula I** may be prepared as shown in Scheme 1 via the coupling of **A** and **B** employing HATU (*N*,*N*,*N'*,*N'*-tetramethyl-*O*-(7-azabenzotriazol-1-yl)uronium hexafluorophosphate) in the presence of DIEA (*N*,N'-diisopropylethylamine).

Deuterated intermediates of type **A** (scheme 1) may be prepared as outlined in scheme 2, analogously to Singh, A.; Van Goor, F.; Worley, F. J. III; Knapp, T. Compounds Useful in CFTR Assays and Methods Therewith WO 2007075946 A1 July 5, 2007.

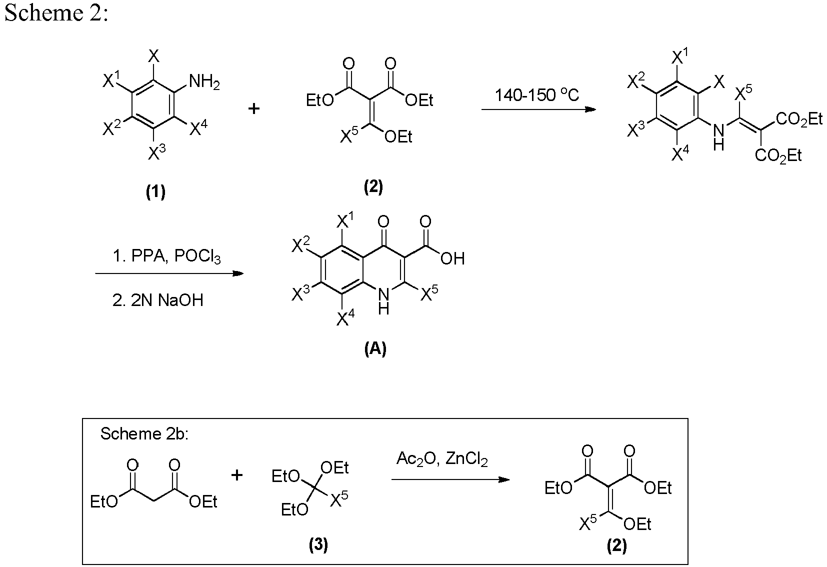

As shown in Scheme 2, heating a mixture of an aniline **1** in the presence of a malonate derivative **2** affords the appropriately deuterated ((phenylamino)methylene)malonate, subsequent exposure of which to polyphosphoric acid in the presence of POCl₃ followed by ester hydrolysis provides carboxylic acid **A.** In Scheme 2, X is hydrogen or deuterium. In one embodiment, X, X¹, X², X³ and X⁴ are the same.

Exemplary compounds for use in Scheme 2 include the embodiment of compound **(1)** where X, X¹, X², X³ and X⁴ are each deuterium, commercially available from Aldrich; and the embodiment of compound **(2)** where X⁵ is deuterium, prepared analogously to the procedure described in Scheme 2b (Parham, W. E.; Reed, L. J. Org. Syn., 1948, 28, 60) for the case where X⁵ is hydrogen by employing the embodiment of **3** where X⁵ is deuterium (available from CDN Isotopes). As shown in Scheme 2b, the appropriately deuterated (ethoxymethylene)malonate of type **2** may be prepared by reaction of diethylmalonate with the appropriately deuterated triethylorthoformate of type **3** in the presence of acetic anhydride and facilitated by ZnCl₂.

Deuterated intermediates of type **B** (Scheme 1) may be prepared as outlined in scheme 3, analogously to Singh, A. et al., *supra.*

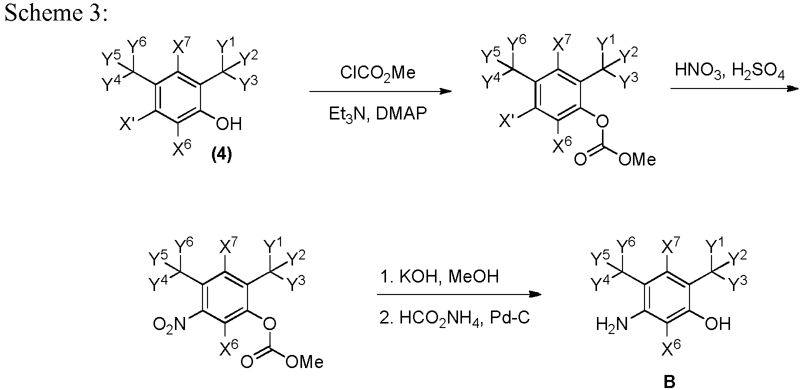

As shown in Scheme 3, protection of ditertbutylphenols of type **4** with methyl chloroformate followed by exposure to nitric acid results in the formation of a nitro-methylcarbonate intermediate. Subsequent carbonate hydrolysis followed by palladium catalyzed reduction of the nitro group ultimately affords aminophenols of type **B.** In Scheme 3, X' is hydrogen or deuterium. In one embodiment, X', X⁶ and X⁷ are the same.

Deuterated intermediates of type **4** (Scheme 3) may be prepared as outlined in Schemes 4a-4d, analogously to Sun, Y.; Tang, N. Huaxue Shiji 2004, 26, 266-268.

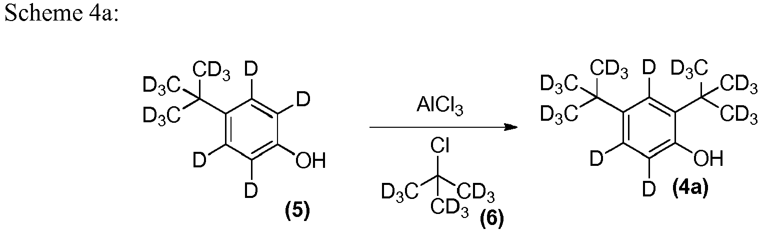

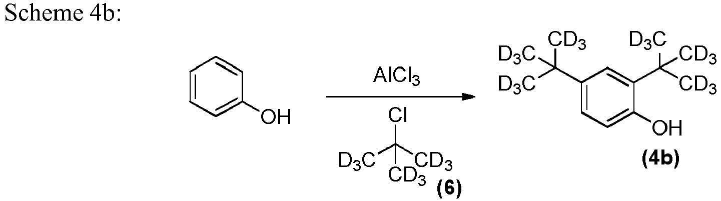

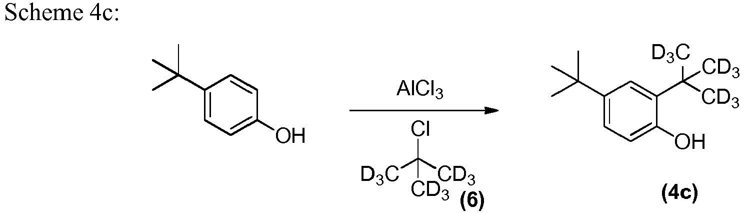

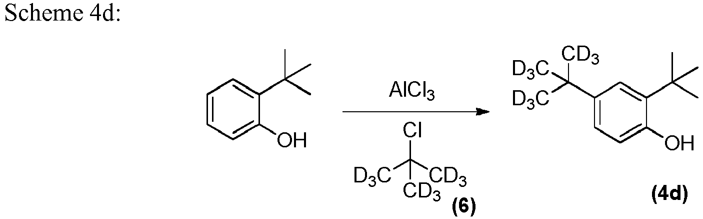

As shown in Schemes 4a-4d, ditertbutylphenols of type **4** may be prepared via Friedel-Crafts alkylation of the appropriately deuterated phenol (phenol, 4-*tert*-butyl phenol or 2-*tert* butylphenol) with d9-*tert* butylchloride. The embodiments of compound **(4)** that may be obtained as shown in scheme 4 are exemplary compounds for use in Scheme 3. In the embodiments **4a, 4b, 4c,** and **4d** in Scheme 4, any atom not designated as deuterium is present at its natural isotopic abundance. In Scheme 4, both Compound **5** and compound **6** are commercially available (CDN Isotopes).

The specific approaches and compounds shown above are not intended to be limiting. The chemical structures in the schemes herein depict variables that are hereby defined commensurately with chemical group definitions (moieties, atoms, etc.) of the corresponding position in the compound formulae herein, whether identified by the same variable name (i.e., R¹, R², R³, etc.) or not. The suitability of a chemical group in a compound structure for use in the synthesis of another compound is within the knowledge of one of ordinary skill in the art.

Additional methods of synthesizing compounds of Formula I and their synthetic precursors, including those within routes not explicitly shown in schemes herein, are within the means of chemists of ordinary skill in the art. Synthetic chemistry transformations and protecting group methodologies (protection and deprotection) useful in synthesizing the applicable compounds are known in the art and include, for example, those described in Larock R, Comprehensive Organic Transformations, VCH Publishers (1989); Greene, TW et al., Protective Groups in Organic Synthesis, 3rd Ed., John Wiley and Sons (1999); Fieser, L et al., Fieser and Fieser's Reagents for Organic Synthesis, John Wiley and Sons (1994); and Paquette, L, ed., Encyclopedia of Reagents for Organic Synthesis, John Wiley and Sons (1995) and subsequent editions thereof.

Combinations of substituents and variables envisioned by this invention are only those that result in the formation of stable compounds.

### Compositions

The invention provides pharmaceutical compositions comprising an effective amount of a compound of Formula I (defined in claim 1), or a pharmaceutically acceptable salt of said compound; and a pharmaceutically acceptable carrier. The carrier(s) are "acceptable" in the sense of being compatible with the other ingredients of the formulation and, in the case of a pharmaceutically acceptable carrier, not deleterious to the recipient thereof in an amount used in the medicament.

Pharmaceutically acceptable carriers, adjuvants and vehicles that may be used in the pharmaceutical compositions of this invention include, but are not limited to, ion exchangers, alumina, aluminum stearate, lecithin, serum proteins, such as human serum albumin, buffer substances such as phosphates, glycine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium chloride, zinc salts, colloidal silica, magnesium trisilicate, polyvinyl pyrrolidone, cellulose-based substances, polyethylene glycol, sodium carboxymethylcellulose, polyacrylates, waxes, polyethylene-polyoxypropylene-block polymers, polyethylene glycol and wool fat.

If required, the solubility and bioavailability of the compounds in pharmaceutical compositions of the invention may be enhanced by methods well-known in the art. One method includes the use of lipid excipients in the formulation. See "Oral Lipid-Based Formulations: Enhancing the Bioavailability of Poorly Water-Soluble Drugs (Drugs and the Pharmaceutical Sciences)," David J. Hauss, ed. Informa Healthcare, 2007; and "Role of Lipid Excipients in Modifying Oral and Parenteral Drug Delivery: Basic Principles and Biological Examples," Kishor M. Wasan, ed. Wiley-Interscience, 2006.

Another known method of enhancing bioavailability is the use of an amorphous form of a compound of this invention optionally formulated with a poloxamer, such as LUTROL™ and PLURONIC™ (BASF Corporation), or block copolymers of ethylene oxide and propylene oxide. See United States patent 7,014,866; and United States patent publications 20060094744 and 20060079502.

The pharmaceutical compositions of the invention are suitable for oral administration. They may also be suitable for rectal, nasal, topical (including buccal and sublingual), vaginal or parenteral (including subcutaneous, intramuscular, intravenous and intradermal) administration. In certain embodiments, the compound of the formulae herein is administered transdermally (e.g., using a transdermal patch or iontophoretic techniques). Other formulations may conveniently be presented in unit dosage form, e.g., tablets, sustained release capsules, and in liposomes, and may be prepared by any methods well known in the art of pharmacy. See, for example, Remington: The Science and Practice of Pharmacy, Lippincott Williams & Wilkins, Baltimore, MD (20th ed. 2000).

Such preparative methods include the step of bringing into association with the molecule to be administered ingredients such as the carrier that constitutes one or more accessory ingredients. In general, the compositions are prepared by uniformly and intimately bringing into association the active ingredients with liquid carriers, liposomes or finely divided solid carriers, or both, and then, if necessary, shaping the product.

In certain embodiments, the compound is administered orally. Compositions of the present invention suitable for oral administration may be presented as discrete units such as capsules, sachets, or tablets each containing a predetermined amount of the active ingredient; a powder or granules; a solution or a suspension in an aqueous liquid or a non-aqueous liquid; an oil-in-water liquid emulsion; a water-in-oil liquid emulsion; packed in liposomes; or as a bolus, etc. Soft gelatin capsules can be useful for containing such suspensions, which may beneficially increase the rate of compound absorption.

In the case of tablets for oral use, carriers that are commonly used include lactose and corn starch. Lubricating agents, such as magnesium stearate, are also typically added. For oral administration in a capsule form, useful diluents include lactose and dried cornstarch. When aqueous suspensions are administered orally, the active ingredient is combined with emulsifying and suspending agents. If desired, certain sweetening and/or flavoring and/or coloring agents may be added.

Compositions suitable for oral administration include lozenges comprising the ingredients in a flavored basis, usually sucrose and acacia or tragacanth; and pastilles comprising the active ingredient in an inert basis such as gelatin and glycerin, or sucrose and acacia.

Compositions suitable for parenteral administration include aqueous and non-aqueous sterile injection solutions which may contain anti-oxidants, buffers, bacteriostats and solutes which render the formulation isotonic with the blood of the intended recipient; and aqueous and non-aqueous sterile suspensions which may include suspending agents and thickening agents. The formulations may be presented in unit-dose or multi-dose containers, for example, sealed ampules and vials, and may be stored in a freeze dried (lyophilized) condition requiring only the addition of the sterile liquid carrier, for example water for injections, immediately prior to use. Extemporaneous injection solutions and suspensions may be prepared from sterile powders, granules and tablets.

Such injection solutions may be in the form, for example, of a sterile injectable aqueous or oleaginous suspension. This suspension may be formulated according to techniques known in the art using suitable dispersing or wetting agents (such as, for example, Tween 80) and suspending agents. The sterile injectable preparation may also be a sterile injectable solution or suspension in a non-toxic parenterally-acceptable diluent or solvent, for example, as a solution in 1,3-butanediol. Among the acceptable vehicles and solvents that may be employed are mannitol, water, Ringer's solution and isotonic sodium chloride solution. In addition, sterile, fixed oils are conventionally employed as a solvent or suspending medium. For this purpose, any bland fixed oil may be employed including synthetic mono- or diglycerides. Fatty acids, such as oleic acid and its glyceride derivatives are useful in the preparation of injectables, as are natural pharmaceutically-acceptable oils, such as olive oil or castor oil, especially in their polyoxyethylated versions. These oil solutions or suspensions may also contain a long-chain alcohol diluent or dispersant.

The pharmaceutical compositions of this invention may be administered in the form of suppositories for rectal administration. These compositions can be prepared by mixing a compound of this invention with a suitable non-irritating excipient which is solid at room temperature but liquid at the rectal temperature and therefore will melt in the rectum to release the active components. Such materials include, but are not limited to, cocoa butter, beeswax and polyethylene glycols.

The pharmaceutical compositions of this invention may be administered by nasal aerosol or inhalation. Such compositions are prepared according to techniques well-known in the art of pharmaceutical formulation and may be prepared as solutions in saline, employing benzyl alcohol or other suitable preservatives, absorption promoters to enhance bioavailability, fluorocarbons, and/or other solubilizing or dispersing agents known in the art. See, e.g.: Rabinowitz JD and Zaffaroni AC, US Patent 6,803,031, assigned to Alexza Molecular Delivery Corporation.

Topical administration of the pharmaceutical compositions of this invention is especially useful when the desired treatment involves areas or organs readily accessible by topical application. For topical application topically to the skin, the pharmaceutical composition should be formulated with a suitable ointment containing the active components suspended or dissolved in a carrier. Carriers for topical administration of the compounds of this invention include, but are not limited to, mineral oil, liquid petroleum, white petroleum, propylene glycol, polyoxyethylene polyoxypropylene compound, emulsifying wax, and water. Alternatively, the pharmaceutical composition can be formulated with a suitable lotion or cream containing the active compound suspended or dissolved in a carrier. Suitable carriers include, but are not limited to, mineral oil, sorbitan monostearate, polysorbate 60, cetyl esters wax, cetearyl alcohol, 2-octyldodecanol, benzyl alcohol, and water. The pharmaceutical compositions of this invention may also be topically applied to the lower intestinal tract by rectal suppository formulation or in a suitable enema formulation. Topically-transdermal patches and iontophoretic administration are also included in this invention.

Application of the subject therapeutics may be local, so as to be administered at the site of interest. Various techniques can be used for providing the subject compositions at the site of interest, such as injection, use of catheters, trocars, projectiles, pluronic gel, stents, sustained drug release polymers or other device which provides for internal access.

In another embodiment, a composition of this invention further comprises a second therapeutic agent. The second therapeutic agent may be selected from any compound or therapeutic agent known to have or that demonstrates advantageous properties when administered with a compound having the same mechanism of action as VX-770.

Preferably, the second therapeutic agent is an agent useful in the treatment of a variety of conditions, including cystic fibrosis, Hereditary emphysema, Hereditary hemochromatosis, Coagulation-Fibrinolysis deficiencies, such as Protein C deficiency, Type 1 hereditary angioedema, Lipid processing deficiencies, such as Familial hypercholesterolemia, Type 1 chylomicronemia, Abetalipoproteinemia, Lysosomal storage diseases, such as I-cell disease/Pseudo-Hurler, Mucopolysaccharidoses, Sandhof/Tay-Sachs, Crigler-Najjar type II, Polyendocrinopathy/Hyperinsulemia, Diabetes mellitus, Laron dwarfism, Myleoperoxidase deficiency, Primary hypoparathyroidism, Melanoma, Glycanosis CDG type 1, Hereditary emphysema, Congenital hyperthyroidism, Osteogenesis imperfecta, Hereditary hypofibrinogenemia, ACT deficiency, Diabetes insipidus (DI), Neurophyseal DI, Neprogenic DI, Charcot-Marie Tooth syndrome, Perlizaeus-Merzbacher disease, neurodegenerative diseases such as Alzheimer's disease, Parkinson's disease, Amyotrophic lateral sclerosis, Progressive supranuclear palsy, Pick's disease, several polyglutamine neurological disorders a such as Huntington, Spinocerebullar ataxia type I, Spinal and bulbar muscular atrophy, Dentatorubal pallidoluysian, and Myotonic dystrophy, as well as Spongiform encephalopathies, such as Hereditary Creutzfeldt-Jakob disease, Fabry disease, Straussler- Scheinker syndrome, COPD, dry-eye disease, and Sjogren's disease.

In one embodiment, the second therapeutic agent is an agent useful in the treatment of cystic fibrosis.

In one embodiment, the second therapeutic agent is VX-809.

In another embodiment, the invention provides separate dosage forms of a compound of this invention and one or more of any of the above-described second therapeutic agents, wherein the compound and second therapeutic agent are associated with one another. The term "associated with one another" as used herein means that the separate dosage forms are packaged together or otherwise attached to one another such that it is readily apparent that the separate dosage forms are intended to be sold and administered together (within less than 24 hours of one another, consecutively or simultaneously).

In the pharmaceutical compositions of the invention, the compound of Formula I is present in an effective amount. As used herein, the term "effective amount" refers to an amount which, when administered in a proper dosing regimen, is sufficient to treat the target disorder.

The interrelationship of dosages for animals and humans (based on milligrams per meter squared of body surface) is described in Freireich et al., Cancer Chemother. Rep, 1966, 50: 219. Body surface area may be approximately determined from height and weight of the subject. See, e.g., Scientific Tables, Geigy Pharmaceuticals, Ardsley, N.Y., 1970,537.

In one embodiment, an effective amount of a compound of Formula I can range from about 0.02 to 2500 mg per treatment. In more specific embodiments the range is from about 0.2 to 1250 mg or from about 0.4 to 500 mg or most specifically from 2 to 250 mg per treatment. Treatment typically is administered one to two times daily.

Effective doses will also vary, as recognized by those skilled in the art, depending on the diseases treated, the severity of the disease, the route of administration, the sex, age and general health condition of the subject, excipient usage, the possibility of co-usage with other therapeutic treatments such as use of other agents and the judgment of the treating physician.

For pharmaceutical compositions that comprise a second therapeutic agent, an effective amount of the second therapeutic agent is between about 20% and 100% of the dosage normally utilized in a monotherapy regime using just that agent. Preferably, an effective amount is between about 70% and 100% of the normal monotherapeutic dose. The normal monotherapeutic dosages of these second therapeutic agents are well known in the art. *See,* e.g., Wells et al., eds., Pharmacotherapy Handbook, 2nd Edition, Appleton and Lange, Stamford, Conn. (2000); PDR Pharmacopoeia, Tarascon Pocket Pharmacopoeia 2000, Deluxe Edition, Tarascon Publishing, Loma Linda, Calif. (2000).

It is expected that some of the second therapeutic agents referenced above will act synergistically with the compounds of Formula I. When this occurs, it will allow the effective dosage of the second therapeutic agent and/or the compound of Formula I to be reduced from that required in a monotherapy. This has the advantage of minimizing toxic side effects of either the second therapeutic agent of a compound of Formula I, synergistic improvements in efficacy, improved ease of administration or use and/or reduced overall expense of compound preparation or formulation.

### Methods of Treatment

Disclosed herein is a method of potentiating the activity of CFTR in an infected cell, comprising contacting such a cell with a compound of Formula I herein, or a pharmaceutically acceptable salt thereof.

Disclosed herein is a method of treating a disease that is beneficially treated by VX-770 in a subject in need thereof, comprising the step of administering to the subject an effective amount of a compound or a composition disclosed herein. In one embodiment the subject is a patient in need of such treatment. Such diseases include cystic fibrosis, Hereditary emphysema, Hereditary hemochromatosis, Coagulation-Fibrinolysis deficiencies, such as Protein C deficiency, Type 1 hereditary angioedema, Lipid processing deficiencies, such as Familial hypercholesterolemia, Type 1 chylomicronemia, Abetalipoproteinemia, Lysosomal storage diseases, such as I-cell disease/Pseudo-Hurler, Mucopolysaccharidoses, Sandhof/Tay-Sachs, Crigler-Najjar type II, Polyendocrinopathy/Hyperinsulemia, Diabetes mellitus, Laron dwarfism, Myleoperoxidase deficiency, Primary hypoparathyroidism, Melanoma, Glycanosis CDG type 1, Hereditary emphysema, Congenital hyperthyroidism, Osteogenesis imperfecta, Hereditary hypofibrinogenemia, ACT deficiency, Diabetes insipidus (DI), Neurophyseal DI, Neprogenic DI, Charcot-Marie Tooth syndrome, Perlizaeus-Merzbacher disease, neurodegenerative diseases such as Alzheimer's disease, Parkinson's disease, Amyotrophic lateral sclerosis, Progressive supranuclear palsy, Pick's disease, several polyglutamine neurological disorders such as Huntington, Spinocerebullar ataxia type I, Spinal and bulbar muscular atrophy, Dentatorubal pallidoluysian, and Myotonic dystrophy, as well as Spongiform encephalopathies, such as Hereditary Creutzfeldt-Jakob disease, Fabry disease, Straussler- Scheinker syndrome, COPD, dry-eye disease, and Sjogren's disease. The invention provides a pharmaceutical composition as defined in the claims for use in treating cystic fibrosis. The invention also provides a pharmaceutical composition as defined in the claims for use in treating chronic obstructive pulmonary disease (COPD).

The method disclosed herein can be used to treat cystic fibrosis in a subject such as a patient in need thereof.

In another embodiment, the treating of cystic fibrosis or COPD according to the invention further comprises the step of co-administering to the subject in need thereof one or more second therapeutic agents. The choice of second therapeutic agent may be made from any second therapeutic agent known to be useful for co-administration with VX-770. The choice of second therapeutic agent is also dependent upon the particular disease or condition to be treated. Examples of second therapeutic agents that may be employed in the treatment are those set forth above for use in combination compositions comprising a compound of Formula I and a second therapeutic agent.

In particular, the combination therapies of this invention include co-administering a compound of Formula I or a pharmaceutically acceptable salt thereof and a second therapeutic agent to a subject in need thereof for treatment of the following conditions (with the particular second therapeutic agent indicated in parentheses following the indication):.

The term "co-administered" as used herein means that the second therapeutic agent may be administered together with a compound of Formula I as part of a single dosage form (such as a composition of this invention comprising a compound of Formula I and an second therapeutic agent as described above) or as separate, multiple dosage forms. Alternatively, the additional agent may be administered prior to, consecutively with, or following the administration of a compound of Formula I. In such combination therapy treatment, both the compounds of this invention and the second therapeutic agent(s) are administered by conventional methods. The administration of a composition of this invention, comprising both a compound of Formula I and a second therapeutic agent, to a subject does not preclude the separate administration of that same therapeutic agent, any other second therapeutic agent or any compound of Formula I to said subject at another time during a course of treatment.

Effective amounts of these second therapeutic agents are well known to those skilled in the art and guidance for dosing may be found in patents and published patent applications referenced herein, as well as in Wells et al., eds., Pharmacotherapy Handbook, 2nd Edition, Appleton and Lange, Stamford, Conn. (2000); PDR Pharmacopoeia, Tarascon Pocket Pharmacopoeia 2000, Deluxe Edition, Tarascon Publishing, Loma Linda, Calif. (2000), and other medical texts. However, it is well within the skilled artisan's purview to determine the second therapeutic agent's optimal effective-amount range.

In one embodiment of the invention, where a second therapeutic agent is administered to a subject, the effective amount of the compound of Formula I is less than its effective amount would be where the second therapeutic agent is not administered. In another embodiment, the effective amount of the second therapeutic agent is less than its effective amount would be where the compound of Formula I is not administered. In this way, undesired side effects associated with high doses of either agent may be minimized. Other potential advantages (including without limitation improved dosing regimens and/or reduced drug cost) will be apparent to those of skill in the art.

Disclosed herein is the use of a compound of Formula I, or a pharmaceutically acceptable salt thereof, alone or together with one or more of the above-described second therapeutic agents in the manufacture of a medicament, either as a single composition or as separate dosage forms, for treatment or prevention in a subject of a disease, disorder or symptom set forth above. Another aspect of the disclosure is a compound of Formula I, or a pharmaceutically acceptable salt thereof, for use in the treatment or prevention in a subject of a disease, disorder or symptom thereof delineated herein.

### Examples

### Example 1. Synthesis of N-(2,-Di-(tert-butyl-d₉)-3,6-d₂-5-hydroxyphenyl)-4-oxo-1,4-dihydroquinoline-3-carboxamide (Compound 110).

Compound **110** was prepared as outlined in Scheme 5 below.

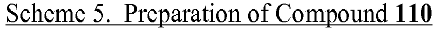

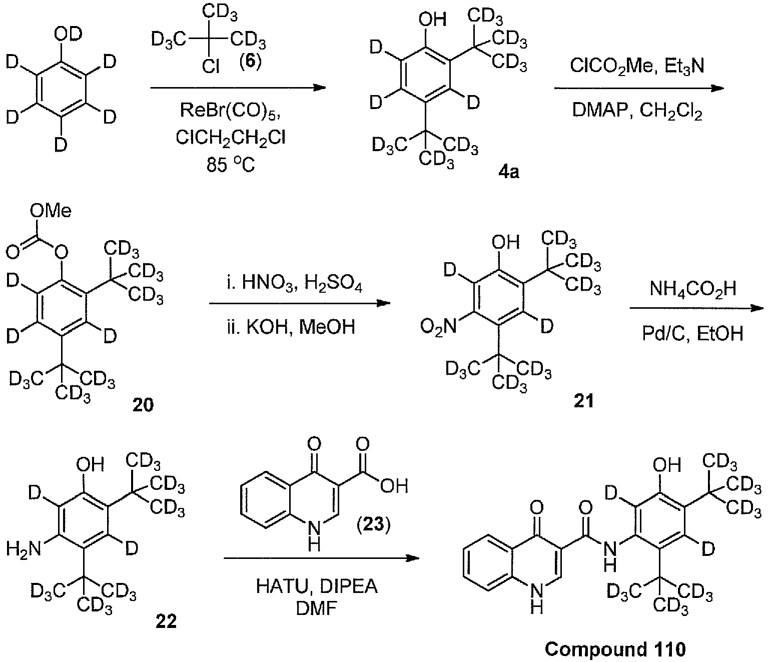

Step 1. 2,4-Di-(*tert*-butyl- d₉)-3,5,6-d₃-phenol (**4a**). Intermediate **4a** was prepared according to the procedure described for the synthesis for 2,4-di-*tert*-butyl-3,5-d₂-phenol employing *tert*-butyl chloride-d₉ in place of *tert*-butylchloride (Kurahashi, T.; Hada, M.; Fujii, H. J. Am. Chem. Soc. 2009, 131, 12394-12405): To a solution of phenol-d₆ (459 mg, 4.59 mmol, 99 atom %D, Sigma Aldrich) and *tert*-butyl chloride-d₉ (2.50 mL, 23.0 mmol, 98 atom%D, Cambridge Isotope Laboratories, Inc.) in 1,2-dichloroethane (10.0 mL) was added ReBr(CO)₅ (19.0 mg, 0.0459 mmol). The reaction mixture was stirred at 85 °C for 15 hours at which time additional *tert*-butyl chloride-dg (2.50 mL, 23.0 mmol, 98 atom%D, Cambridge Isotope Laboratories, Inc.) and ReBr(CO)₅ (19.0 mg, 0.0459 mmol) was added. Stirring was continued at 85 °C for 2 hours, the mixture was cooled to room temperature, concentrated *in vacuo* and purified by column chromatography (SiO₂, 30% CH₂Cl₂/heptanes) to afford **4a** (0.789 g, 76% yield) as a light yellow oil. MS (ESI) 228.1 [(M+H)⁺].

Step 2. 2,4-Di-(tert-bulyl-d₉)-3,5,6-d₃-phenyl methyl carbonate (**20)**. To a solution of **4a** (2.72 g, 12.0 mmol), triethylamine (3.33 mL, 23.9 mmol) and *N,N-*dimethylaminopyridine (73.0 mg, 0.598 mmol) in CH₂Cl₂ (30.0 mL) at 0 °C was added methyl chloroformate (1.38 mL, 17.9 mmol). The reaction mixture was stirred at room temperature for 15 hours then was diluted with 10% ethyl acetate/heptanes and filtered through a silica plug. The silica plug was then rinsed with additional 10% ethyl acetate/heptanes. The filtrate was combined, and concentrated *in vacuo* to provide **20** (2.40 g, 70% yield) as a light yellow oil which was carried forward without purification.

Step 3. 2,4-Di-(tert-butyl-d₉)-3,6-d₂-5-nitrophenol (**21**). To a solution of **20** (2.40 g, 8.41 mmol) in sulfuric acid (1.00 mL) at 0 °C was added a 1:1 mixture of sulfuric acid and nitric acid (2.00 mL) dropwise. The reaction mixture was then stirred at room temperature for 2 hours then slowly added to ice water with vigorous stirring. The resulting slurry was extracted with ethyl acetate (3 x 100mL) and the combined organic layers were dried (Na₂SO₄), filtered and concentrated to afford an amber oil containing a mixture of regioisomers. This crude oil was then taken up in MeOH (50 mL) and KOH (1.54 g, 27.5 mmol) was added. The reaction mixture was stirred at room temperature for 2 hours then was acidified to pH = 2 with concentrated HCl. The resulting solution was extracted with diethyl ether (3 x 100 mL), dried (MgSO₄), filtered and concentrated. The residue was then purified via column chromatography (SiO₂, 0-5% ethyl acetate/heptanes) to afford **21** (526 mg, 23%) as a light yellow solid. MS (ESI) 270.3 [(M-H)⁻].

Step 4. 5-Amino-2,4-di-(tert-butyl-d₉)-3,6-d₂-phenol (**22**). A solution of **21** (526 mg, 1.94 mmol) and ammonium formate (489 mg, 7.75 mmol) in ethanol (25.0 mL) was heated to the point of reflux. At this time, 10% Pd/C (250 mg, 50% wet) was added in small portions and the reaction mixture was stirred at reflux for 2 hours. The mixture was then cooled to room temperature, diluted with THF, filtered through Celite® and concentrated *in vacuo* to afford **22** (473 mg, 100%) as a tan solid. MS (ESI) 242.4 [(M+H)⁺].

Step 5. N-(2,4-Di-(tert-butyl-d₉)-3,6-d₂-5-hydroxyphenyl)-4-oxo-1,4-dihvdroquinoline-3-carboxamide (Compound **110**). To a solution of **22** (250 mg, 1.04 mmol), 4-oxo-1,4-dihydroquinoline-3-carboxylic acid (**23,** purchased from Matrix Scientific, 98.0 mg, 0.518 mmol) and N,N-diisopropylethylamine (181 µL, 1.04 mmol) in DMF (5.00 mL) was added HATU (197 mg, 0.518 mmol). The reaction mixture was stirred at room temperature for 3 hours then was diluted with saturated NaHCO₃ and extracted with ethyl acetate (3 x 50 mL). The combined organic extracts were washed with water (3 x 20 mL), dried (Na₂SO₄), filtered and concentrated *in vacuo.* The resulting residue was purified via column chromatography (SiO₂, 0-70% ethyl acetate/heptanes) to afford Compound **110** (77.0 mg, 36% Yield) as a white solid. ¹H NMR (d₆-DMSO, 400 MHz): δ 12.87 (br s, 1H), 11.80 (s, 1H), 9.18 (s, 1H), 8.86 (s, 1H), 8.32 (d, *J* = 8.2 Hz, 1H), 7.81 (t, *J* = 7.9 Hz, 1H), 7.76 (t, *J* = 8.2 Hz, 1H), 7.51 (t, *J* = 7.4 Hz, 1H), 7.10 (s, 0.2H)*; MS (ESI) 413.5 [(M+H)⁺]. *The ¹H NMR signal at 7.10 ppm indicates approximately 80% deuterium incorporation at one of the two deuterated aryl positions. The absence of signals at 7.20 ppm and 1.37 ppm indicate high levels of incorporation (>95%) at the remaining deuterated positions.

### Example 2. Evaluation of Metabolic Stability - Human CYP3A4 Supersomes™.

*SUPERSOMES™ Assay.* 7.5 mM stock solutions of test compounds, Compound **110** and ivacaftor, were prepared in DMSO. The 7.5 mM stock solutions were diluted to 50 mM in acetonitrile (ACN). Human CYP3A4 supersomes™ (1000 pmol/mL, purchased from BD Gentest™ Products and Services) were diluted to 62.5 pmol/mL in 0.1 M potassium phosphate buffer, pH 7.4, containing 3 mM MgCl₂. The diluted supersomes were added to wells of a 96-well polypropylene plate in triplicate. A 10 mL aliquot of the 50 mM test compound was added to the supersomes and the mixture was pre-warmed for 10 minutes. Reactions were initiated by addition of pre-warmed NADPH solution. The final reaction volume was 0.5 mL and contained 50 pmol/mL CYP3A4 supersomes™, 1.0 mM test compound, and 2 mM NADPH in 0.1 M potassium phosphate buffer, pH 7.4, and 3 mM MgCl₂. The reaction mixtures were incubated at 37 °C, and 50 mL aliquots were removed at 0, 5, 10, 20, and 30 minutes and added to 96-well plates which contained 50 mL of ice-cold ACN with internal standard to stop the reactions. The plates were stored at 4 °C for 20 minutes after which 100 mL of water was added to the wells of the plate before centrifugation to pellet precipitated proteins. Supernatants were transferred to another 96-well plate and analyzed for amounts of parent remaining by LC-MS/MS using an Applied Bio-systems API 4000 mass spectrometer.

***Data analysis:*** The *in vitro* half-lives (t_{1/2} values) for test compounds were calculated from the slopes of the linear regression of LN(% parent remaining) vs incubation time relationship:
*in vitro* t _{1/2} = 0.693/k, where k = -[slope of linear regression of % parent remaining (In) vs incubation time]
Figure 1 shows a plot of the percentage of parent compound remaining over time for Compound **110** and for ivacaftor in human cytochrome P450-specific SUPERSOMES™.The t_{1/2} values, and the percentage increase (% Δ) in average t_{1/2}, are shown in Table 4 below.

**Table 4. Results of In Vitro Human Cytochrome P450-Specific SUPERSOMES™**

| | **t_{1/2} (min)** | | | |
|---|---|---|---|---|
| **Compound** | ***Experiment 1*** | ***Experiment 2*** | ***Experiment 3*** | **AVE± SD % Δ** |
| Ivacaftor | 5.2 | 6.0 | 5.8 | 5.7±0.4 |
| Compound **110** | 9.9 | 7.9 | 8.7 | 8.8±0.8 **55%** |

Table 4 shows that Compound **110** has a 55% longer half life in the assay than ivacaftor.

The subject matter of the invention is set out in the following claims.

| | | | | | | | | | | | | | |
|---|---|---|---|---|---|---|---|---|---|---|---|---|---|
| 122 | H | H | H | H | H | H | D | CD₃ | CD₃ | CD₃ | CH₃ | CH₃ | CH₃ |

## Claims

1. A pharmaceutical composition suitable for oral administration comprising a compound of Formula I: 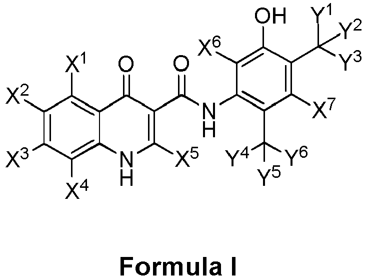 or a pharmaceutically acceptable salt thereof, wherein
each of X¹, X², X³, X⁴, X⁵, X⁶, and X⁷ is independently hydrogen or deuterium;
Y¹, Y², and Y³ are CD₃;
each of Y⁴, Y⁵, and Y⁶ is independently CH₃ or CD₃; wherein the isotopic enrichment factor for each designated deuterium atom is at least 6000; and wherein any atom not designated as deuterium is present at its natural isotopic abundance, provided that the compound of Formula I is not compound 100: 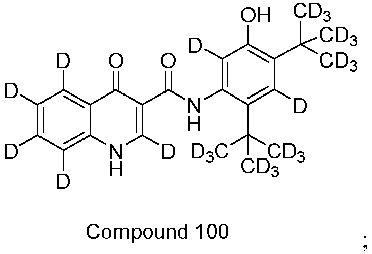 and a pharmaceutically acceptable carrier.

2. The pharmaceutical composition of claim 1, wherein the pharmaceutical composition is in unit dosage form.

3. The pharmaceutical composition of claim 2, wherein the unit dosage form is a tablet.

4. The pharmaceutical composition of any one of the preceding claims, further comprising a second therapeutic agent useful in the treatment of cystic fibrosis or chronic obstructive pulmonary disease (COPD).

5. The pharmaceutical composition of any of the preceding claims, wherein X¹, X², X³, and X⁴ of the compound of Formula I are the same.

6. The pharmaceutical composition of claim 5, wherein Y⁴, Y⁵, and Y⁶ of the compound of Formula I are the same.

7. The pharmaceutical composition of any one of claims 5 to 6, wherein X⁵ of the compound of Formula I is hydrogen.

8. The pharmaceutical composition of any one of claims 5 to 6, wherein Y⁴, Y⁵, and Y⁶ of the compound of Formula I are CH₃.

9. The pharmaceutical composition of claim 1, wherein the pharmaceutical composition is suitable for oral administration.

10. The pharmaceutical composition of claim 1, wherein the isotopic enrichment factor for each designated deuterium atom of the compound of Formula I is at least 6333.3 or at least 6466.7.

11. The pharmaceutical composition of claim 1 or claim 10, wherein the compound of Formula I is any one of the compounds of the table below,
| **Cmpd #** | **X¹** | **X²** | **X³** | **X⁴** | **X⁵** | **X⁶** | **X⁷** | **Y¹** | **Y²** | **Y³** | **Y⁴** | **Y⁵** | **Y⁶** |
|---|---|---|---|---|---|---|---|---|---|---|---|---|---|
| | | | | | | | | | | | | | |
| 101 | H | H | H | H | D | H | H | CD₃ | CD₃ | CD₃ | CD₃ | CD₃ | CD₃ |
| 102 | H | H | H | H | D | H | H | CD₃ | CD₃ | CD₃ | CH₃ | CH₃ | CH₃ |
| 105 | H | H | H | H | H | H | H | CD₃ | CD₃ | CD₃ | CD₃ | CD₃ | CD₃ |
| 106 | H | H | H | H | H | H | H | CD₃ | CD₃ | CD₃ | CH₃ | CH₃ | CH₃ |
or a pharmaceutically acceptable salt thereof.

12. The pharmaceutical composition of claim 1 or claim 10, wherein the compound of Formula I is the compound of the table below,
| **Cmpd #** | **X¹** | **X²** | **X³** | **X⁴** | **X⁵** | **X⁶** | **X⁷** | **Y¹** | **Y²** | **Y³** | **Y⁴** | **Y⁵** | **Y⁶** |
|---|---|---|---|---|---|---|---|---|---|---|---|---|---|
| | | | | | | | | | | | | | |
| 106 | H | H | H | H | H | H | H | CD₃ | CD₃ | CD₃ | CH₃ | CH₃ | CH₃ |
or a pharmaceutically acceptable salt thereof.

13. A pharmaceutical composition of any of the preceding claims for use in treating cystic fibrosis.

14. A pharmaceutical composition of any one of the preceding claims for use in treating chronic obstructive pulmonary disease (COPD).

## Patentansprüche

1. Pharmazeutische Zusammensetzung, die für die orale Verabreichung geeignet ist und eine Verbindung der Formel I: 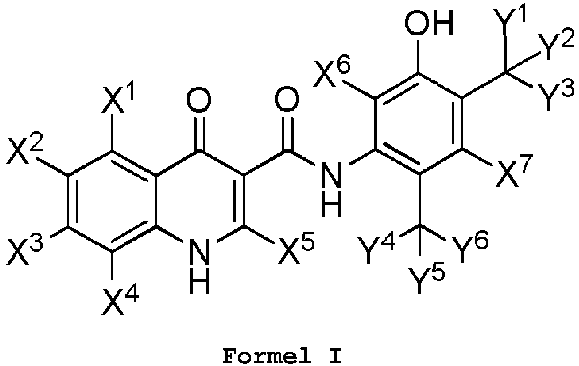 oder ein pharmazeutisch unbedenkliches Salz davon umfasst, wobei
X¹, X², X³, X⁴, X⁵, X⁶ und X⁷ jeweils unabhängig für Wasserstoff oder Deuterium stehen;
Y¹, Y² und Y³ für CD₃ stehen;
Y⁴, Y⁵ und Y⁶ jeweils unabhängig für CH₃ oder CD₃ stehen; wobei der Isotopenanreicherungsfaktor für jedes angegebene Deuteriumatom mindestens 6000 beträgt; und wobei jedes nicht als Deuterium angegebene Atom in seiner natürlichen Isotopenhäufigkeit vorliegt, mit der Maßgabe, dass es sich bei der Verbindung mit der Formel I nicht um Verbindung 100: 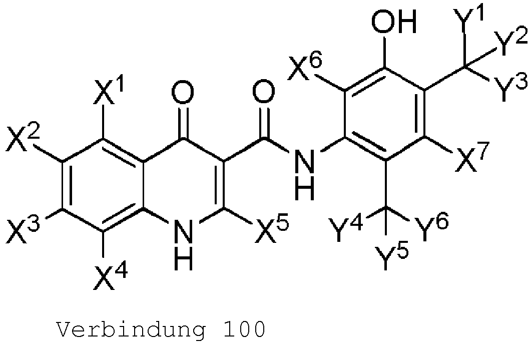 handelt;
und einen pharmazeutisch unbedenklichen Träger.

2. Pharmazeutische Zusammensetzung nach Anspruch 1, wobei die pharmazeutische Zusammensetzung in Dosierungseinheitsform vorliegt.

3. Pharmazeutische Zusammensetzung nach Anspruch 2, wobei es sich bei der Dosierungseinheitsform um eine Tablette handelt.

4. Pharmazeutische Zusammensetzung nach einem der vorhergehenden Ansprüche, ferner umfassend ein zweites therapeutisches Mittel, das zur Verwendung bei der Behandlung von zystischer Fibrose oder chronisch-obstruktiver Lungenerkrankung (COPD) geeignet ist.

5. Pharmazeutische Zusammensetzung nach einem der vorhergehenden Ansprüche, wobei X¹, X², X³ und X⁴ der Verbindung der Formel I gleich sind.

6. Pharmazeutische Zusammensetzung nach Anspruch 5, wobei Y⁴, Y⁵ und Y⁶ der Verbindung der Formel I gleich sind.

7. Pharmazeutische Zusammensetzung nach einem der Ansprüche 5 bis 6, wobei X⁵ der Verbindung der Formel I für Wasserstoff steht.

8. Pharmazeutische Zusammensetzung nach einem der Ansprüche 5 bis 6, wobei Y⁴, Y⁵ und Y⁶ der Verbindung der Formel I für CH₃ stehen.

9. Pharmazeutische Zusammensetzung nach Anspruch 1, wobei die pharmazeutische Zusammensetzung für die orale Verabreichung geeignet ist.

10. Pharmazeutische Zusammensetzung nach Anspruch 1, wobei der Isotopenanreicherungsfaktor für jedes angegebene Deuteriumatom der Verbindung der Formel I mindestens 6333,3 oder mindestens 6466,7 beträgt.

11. Pharmazeutische Zusammensetzung nach Anspruch 1 oder Anspruch 10, wobei es sich bei der Verbindung der Formel I um eine der Verbindungen der nachstehenden Tabelle
| **Verb.Nr.** | **X¹** | **X²** | **X³** | **X⁴** | **X⁵** | **X⁶** | **X⁷** | **Y¹** | **Y²** | **Y³** | **Y⁴** | **Y⁵** | **Y⁶** |
|---|---|---|---|---|---|---|---|---|---|---|---|---|---|
| | | | | | | | | | | | | | |
| 101 | H | H | H | H | D | H | H | CD₃ | CD₃ | CD₃ | CD₃ | CD₃ | CD₃ |
| 102 | H | H | H | H | D | H | H | CD₃ | CD₃ | CD₃ | CH₃ | CH₃ | CH₃ |
| 105 | H | H | H | H | H | H | H | CD₃ | CD₃ | CD₃ | CD₃ | CD₃ | CD₃ |
| 106 | H | H | H | H | H | H | H | CD₃ | CD₃ | CD₃ | CH₃ | CH₃ | CH₃ |
oder ein pharmazeutisch unbedenkliches Salz davon handelt.

12. Pharmazeutische Zusammensetzung nach Anspruch 1 oder Anspruch 10, wobei es sich bei der Verbindung der Formel I um die Verbindung der nachstehenden Tabelle
| **Verb.Nr.** | **X¹** | **X²** | **X³** | **X⁴** | **X⁵** | **X⁶** | **X⁷** | **Y¹** | **Y²** | **Y³** | **Y⁴** | **Y⁵** | **Y⁶** |
|---|---|---|---|---|---|---|---|---|---|---|---|---|---|
| | | | | | | | | | | | | | |
| 106 | H | H | H | H | H | H | H | CD₃ | CD₃ | CD₃ | CH₃ | CH₃ | CH₃ |
oder ein pharmazeutisch unbedenkliches Salz davon handelt.

13. Pharmazeutische Zusammensetzung nach einem der vorhergehenden Ansprüche zur Verwendung bei der Behandlung von zystischer Fibrose.

14. Pharmazeutische Zusammensetzung nach einem der vorhergehenden Ansprüche zur Verwendung bei der Behandlung von chronisch-obstruktiver Lungenerkrankung (COPD).

## Revendications

1. Composition pharmaceutique appropriée pour une administration par voie orale comprenant un composé de formule I : 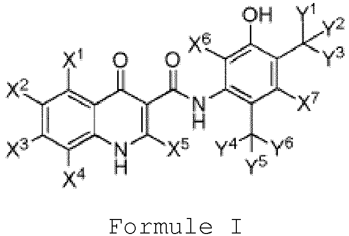 ou un sel pharmaceutiquement acceptable de celui-ci,
chacun de X¹, X², X³, X⁴, X⁵, X⁶ et X⁷ étant indépendamment hydrogène ou deutérium ;
Y¹, Y² et Y³ étant CD₃ ;
chacun de Y⁴, Y⁵ et Y⁶ étant indépendamment CH₃ ou CD₃; le facteur d'enrichissement isotopique pour chaque atome de deutérium indiqué étant d'au moins 6000 ; et chaque atome non désigné comme deutérium étant présent dans son abondance isotopique naturelle, étant entendu que le composé de formule I n'est pas le composé 100 : 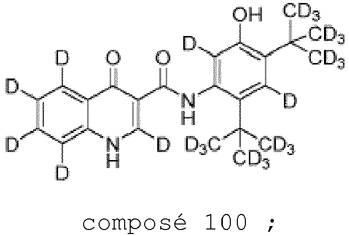 et un véhicule pharmaceutiquement acceptable.

2. Composition pharmaceutique selon la revendication 1, la composition pharmaceutique se trouvant sous forme de dosage unitaire.

3. Composition pharmaceutique selon la revendication 2, la forme de dosage unitaire étant un comprimé.

4. Composition pharmaceutique selon l'une quelconque des revendications précédentes, comprenant en outre un deuxième agent thérapeutique utile dans le traitement de la fibrose cystique ou de la bronchopneumopathie chronique obstructive (BPCO).

5. Composition pharmaceutique selon l'une quelconque des revendications précédentes, X¹, X², X³ et X⁴ du composé de formule I étant identiques.

6. Composition pharmaceutique selon la revendication 5, Y⁴, Y⁵ et Y⁶ du composé de formule I étant identiques.

7. Composition pharmaceutique selon l'une quelconque des revendications 5 et 6, X⁵ du composé de formule I étant hydrogène.

8. Composition pharmaceutique selon l'une quelconque des revendications 5 et 6, Y⁴, Y⁵ et Y⁶ du composé de formule I étant CH₃.

9. Composition pharmaceutique selon la revendication 1, la composition pharmaceutique étant appropriée pour une administration par voie orale.

10. Composition pharmaceutique selon la revendication 1, le facteur d'enrichissement isotopique pour chaque atome de deutérium indiqué du composé de formule I étant d'au moins 6333,3 ou d'au moins 6466,7.

11. Composition pharmaceutique selon la revendication 1 ou la revendication 10, le composé de formule I étant l'un quelconque des composés du tableau ci-dessous,
| Composé n° | X¹ | X² | X³ | X⁴ | X⁵ | X⁶ | X⁷ | Y¹ | Y² | Y³ | Y⁴ | Y⁵ | Y⁶ |
|---|---|---|---|---|---|---|---|---|---|---|---|---|---|
| | | | | | | | | | | | | | |
| 101 | H | H | H | H | D | H | H | CD₃ | CD₃ | CD₃ | CD₃ | CD₃ | CD₃ |
| 102 | H | H | H | H | D | H | H | CD₃ | CD₃ | CD₃ | CH₃ | CH₃ | CH₃ |
| 105 | H | H | H | H | H | H | H | CD₃ | CD₃ | CD₃ | CD₃ | CD₃ | CD₃ |
| 106 | H | H | H | H | H | H | H | CD₃ | CD₃ | CD₃ | CH₃ | CH₃ | CH₃ |
ou un sel pharmaceutiquement acceptable de celui-ci.

12. Composition pharmaceutique selon la revendication 1 ou la revendication 10, le composé de formule I étant le composé du tableau ci-dessous,
| Composé n° | X¹ | X² | X³ | X⁴ | X⁵ | X⁶ | X⁷ | Y¹ | Y² | Y³ | Y⁴ | Y⁵ | Y⁶ |
|---|---|---|---|---|---|---|---|---|---|---|---|---|---|
| | | | | | | | | | | | | | |
| 106 | H | H | H | H | H | H | H | CD₃ | CD₃ | CD₃ | CH₃ | CH₃ | CH₃ |
ou un sel pharmaceutiquement acceptable de celui-ci.

13. Composition pharmaceutique selon l'une quelconque des revendications précédentes pour une utilisation dans le traitement de la fibrose cystique.

14. Composition pharmaceutique selon l'une quelconque des revendications précédentes pour une utilisation dans le traitement de la bronchopneumopathie chronique obstructive (BPCO).
